# EUROPEAN PATENT APPLICATION

(11) **EP 1 964 579 A1**
(43) Date of publication of application: **03.09.2008**
(21) Application number: 07290186.1
(22) Date of filing: 14.02.2007
(51) Int. Cl.: A61L 2/00

(54) **Preparation of virally inactivated biological fluids having high alpha-2-antiplasmin activity**

(71) Applicant: Fondation pour la Recherche Diagnostique, 1785 Cressier s/Murat (CH)
(72) Inventor: Burnouf, Thierry, 59249 Aubers (FR); El-Ekiaby, Magdy, Cairo (EG); Goubran, Hadi Alphonse, Cairo (EG); Radosevich, Miryana, 59249 Aubers (FR)
(74) Representative: Cabinet Plasseraud

(57) **Abstract**

The invention relates to the use of polyoxyethylene (4-5) p-t-octyl phenol (t-Oct-C₆H₄-(OCH₂CH₂)ₓOH, x= -5) or polyoxyethylene (20) sorbitan monooleate in solvent/detergent viral inactivation of biological fluids for the preparation of a virally inactivated biological fluid having a high alpha 2-antiplasmin content.

## Description

The present invention concerns the use of polyoxyethylene (4-5) p-t-octyl phenol or polyoxyethylene (20) sorbitan monooleate for the preparation of a virally inactivated biological fluid having a high alpha-2-antiplasmin (α2-AP) activity.

Human and animal blood plasma and plasma fractions play an important role in modern medicine. They are mainly used for transfusion purposes in the treatment of bleeding episodes, thrombotic episodes, fibrinolysis, infectious episodes, or for prophylaxis or for pretreating patients prior to surgery in certain clinical situations.

A major drawback in the use of human or animal blood plasma or plasma fractions is the risk of transmission of blood borne viruses, such as human immunodeficiency virus (HIV), hepatitis B virus (HBV), hepatitis C virus (HCV), and West Nile Virus, or possibly emerging viruses such as Severe Acute Respiratory Syndrome (SARS) virus, and avian flu virus, which are all lipid-enveloped viruses. Viral risks also exist from products obtained from recombinant mammalian cell lines and from transgenic animals.

Therefore, different methods for viral inactivation of blood plasma and/or plasma fractions and/or recombinant products have been developed, such as the treatment with methylene blue followed by irradiation with visible light and solvent/detergent or solvent alone treatment. US patent Nos. 4,481,189 and 4,540,573 describe for example the use of organic solvent detergent combinations (S/D method) or solvent alone to reduce by several orders of magnitude the infectivity of hepatitis viruses or other enveloped viruses contained in plasma and plasma products or added thereto.

Solvent/detergent or solvent alone treatment under appropriate conditions of reagent concentration, temperature and contact time effectively disassembles viruses that have envelope proteins associated with lipids, while having negligible effect on the molecular conformations and biological activity of sensitive plasma proteins.

Today the solvent/detergent (S/D) method is the main viral inactivation treatment of many plasma and biotech (recombinant or transgenic products derived from mammalian cells or mammals) products on the market, including plasma for transfusion. The procedure generally consists in incubating protein solutions for 1 to 6 hours with 0.3 to 1% by weight with respect to the weight of the protein solution of an organic solvent, tri(n-butyl) phosphate (TnBP), and of one or several detergents, generally Tween-80, Triton X-100, or sodium deoxycholate. The plasma or plasma products are treated in large pools of a large number of donations (100 to 5000 litres, or more), which requires large and expensive pharmaceutical manufacturing facilities.

The treatment preserves the functional activities of a wide range of coagulation factors and other labile plasma proteins with an excellent record of virucidal efficiency for the pathogenic lipid-enveloped blood-borne viruses (Burnouf and Radosevich , Blood Rev 2000, 14: 94-110).

Still, large-pool SD (LP/SD) plasma for transfusion subjected to incubation with 1 weight% of TnBP with respect to the weight of the biological fluid and 1 weight% of Triton X-100 (polyoxyethylene (9-10) p-t-octyl phenol; molecular formula t-Oct-C₆H₄-(OCH₂CH₂)ₓ, x=9-10) with respect to the weight of the biological fluid, oil extraction, centrifugation, and hydrophobic interaction chromatography as for example described in Horowitz B. et al., Blood 1992; 79: 826-31, has low functional activity of certain serine protease inhibitors (serpins), such as alpha 2-antiplasmin (α2-AP or SERPINF2) as evidenced by Hellstern P. et al.. Vox Sang 1992;63: 178-85 and Mast AE, et al, Blood 1999;94: 3922-7, and alpha-1-antitrypsin (α1-AT or SERPINA1) as *evidenced by* Mast AE, et al., Blood 1999;94: 3922-7.

Mast et al. attribute the loss of α2-AP activity to a conversion of the native conformation of the protein to a latent or polymerized inactive form (Mast et al., Blood 1999, 94, 3922-7).

The loss of α2-AP activity has frequently been explained by the denaturation of α2-AP due to the harsh and time-consuming industrial processing steps performed at a large scale, such as high-speed centrifugation, or the chromatography of plasma materials on hydrophobic materials performed for the removal of the SD agents.

Low α2-AP activity is suspected to have contributed to severe hyperfibrinolysis when LP/SD plasma was used during liver transplant, highlighting the potential clinical significance of this depletion (de Jonge et al., Anesth Analg. 2002, 94: 1127-1131).

Recently, the present inventors have developed a solvent/detergent process applied to minipools of plasma (MP/SD). In this process, which is described in international patent application WO 2006/082115, small quantities of plasma are treated in a closed bag system.

After long and intensive research, the inventors have now found surprisingly and unexpectedly that the loss of α2-AP activity in S/D viral inactivation with TnBP and Triton X-100 is not due to the harsh industrial processing steps performed at a large scale, but also occurs when using the recently developed MP/SD process.

It exists therefore a need for virally inactivated plasma having high activity of serine protease inhibitors (serpins), such as alpha-2-antiplasmin (α2-AP or SERPINF2) and alpha-1-antitrypsin (α1-AT or SERPINA1).

One of the objectives of the present invention is thus to provide a means for preparing virally inactivated biologically fluids, such as plasma, plasma products or recombinant or transgenic products, having a high α2-AP activity.

Another objective of the invention is to provide a means for preparing virally inactivated biologically fluids that also have high α1-AT activity.

A further objective of the invention is to provide a means for preparing virally inactivated biologically fluids that also have high plasminogen activator inhibitor-1 activity (PAI-1).

After long and intensive research, the inventors have now found, surprisingly and unexpectedly, that the objective can be reached by using polyoxyethylene (4-5) p-t-octyl phenol (t-Oct-C₆H₄- (OCH₂CH₂)ₓOH, x= ~5), which is commercially available under the name Triton X-45, or polyoxyethylene (20) sorbitan monooleate, which is commercially available under the name Tween 80, as detergent in S/D viral inactivation of biological fluids.

Therefore, the present invention particularly relates to the use of polyoxyethylene (4-5) p-t-octyl phenol (t-Oct-C₆H₄-(OCH₂CH₂)ₓOH, x= ~5) or polyoxyethylene (20) sorbitan monooleate in S/D viral inactivation of biological fluids for the preparation of a virally inactivated biological fluid wherein the virally inactivated biological fluid has an α2-AP activity of at least 85 % of the initial α2-AP content. Preferably, the α2-AP activity is at least 88 %, and even more preferably at least 90 % of the initial α2-AP activity. In a particularly preferred embodiment the α2-AP activity is at least 92 % of the initial α2-AP activity.

In an advantageous embodiment, the virally inactivated biological fluid also has an α1-AT activity of at least 80 %, preferably 85 %, and even more preferably of at least 90 % of the initial α1-AT activity.

Advantageously, the virally inactivated biological fluid also has a PAI-1 activity of at least 80 %, preferably 85 %, and even more preferably of at least 90 % of the initial PAI-1 activity.

A high PAI-1 activity lowers the risks of fibrinolysis and bleeding in some patients.

Advantageously, the polyoxyethylene (4-5) p-t-octyl phenol (t-Oct-C₆H₄(OCH₂CH₂)ₓOH, x= ~5) (Triton x-45) is used in an amount from 0.1 to 2 weight% with respect to the weight of the biological fluid, preferably 0.3 to 1.5 weight%, and even more preferably 0.5 to 1 weight%, for example 1 weight% for the viral inactivation of complex mixtures like plasma, or cryo-poor plasma or 1 weight% for the viral inactivation of more purified fractions like prothrombin complex concentrate, α2-AP, alpha 1-AT, PAI-1, or other protease inhibitor concentrates.

The polyoxyethylene (20) sorbitan monooleate (Tween 80) is adavantageously used in an amount from 0.1 to 2 weight% with respect to the weight of the biological fluid, preferably 0.3 to 1.5 weight%, and even more preferably 0.5 to 1 weight%, for example 1 weight% for the viral inactivation of complex mixtures like plasma, or cryo-poor plasma or 1 weight% for the viral inactivation of more purified fractions like prothombin complex concentrate, α2-AP, alpha 1-AT, PAI-1,, or other protease inhibitor concentrates.

Biological fluids according to the present invention comprise mammalian blood, blood plasma, blood serum, plasma fractions, precipitates from blood fractions and supernatants from blood fractionation, platelet poor plasma, cryo-poor plasma (cryosupernatant), recombinant and transgenic products. Preferred biological fluids are blood plasma, including recovered plasma (plasma from whole blood) and apheresis plasma, cryo-poor plasma, plasma fractions, such as fractions for the purification of prothrombin complex, Factor IX, Factor VII, Protein C, Protein S, Antithrombin, α2-AP, α1-AT, PAI-1,, C1-inhibitor, precipitates from plasma, such as polyethylene glycol, caprylic acid, or ammonium sulphate precipitated fractions, and their corresponding supernatants, or any other precipitates or supernatants from plasma fractionation, such as cryosupernatant, supernatant I+II+III, precipitate I+II+III, supernatant II+III, precipitate II+III, supernatant I+III, supernatant IV. Plasma fractions, precipitates and supernatants may be obtained from recovered plasma (plasma from whole blood) as well as from apheresis plasma.

Suitable solvents for use according to the invention are those conventionally used in S/D viral inactivation of biological fluids and comprise in particular di- or trialkylphosphates, such as tri-(n-butyl)phosphate (TnBP), tri-(t-butyl)phosphate, tri-(n-hexyl)phosphate, tri-(2-ethylhexyl)phosphate, tri-(n-decyl)phosphate, di-(n-butyl)phosphate, di-(t-butyl)phosphate, di-(n-hexyl)phosphate, di-(2-ethylhexyl)phosphate, di-(n-decyl)phosphate as well as dialkylphosphates with different alkyl chains. Di- or trialkylphosphates having different alkyl chains can be employed, for example ethyl di(n-butyl) phosphate. An especially preferred trialkylphosphate is tri-(n-butyl)phosphate (TnBP).

Advantageously, the solvent is used in an amount from 0.1 to 2 weight% with respect to the weight of the biological fluid, preferably 0.3 to 1 weight%, and even more preferably 1 weight% for the viral inactivation of complex mixtures like plasma, cryo-poor plasma , or 0.3 weight% for the viral inactivation of more purified fractions like Factor VIII.

The biological fluid is treated with the solvent/detergent combination during a period of 1 to 8 hours, preferably 1.5 to 6 hours, for example 2 to 4 hours.

The present invention can be applied to conventional LP/SD viral inactivation of biological fluids and to MP/SD viral inactivation of biological fluids as described in WO 2006/082115.

Generally S/D viral inactivation of biological fluids comprises the steps of viral inactivation of the biological fluid with a solvent/detergent preparation, oil extraction of the solvent/detergent containing biological fluid in order to remove solvent and detergent. The oil extraction step can be carried out once or several times, for example two or three times. Optionally, the process may also comprise, after the oil extraction, a step of centrifugation and hydrophobic interaction chromatography (HIC) in order to remove remaining solvent.

The present invention is particular useful in the MP/SD viral inactivation of biological fluids, such as plasma for transfusion, since it ensures that the MP/SD biological fluids will contain active amounts of α2-AP, alpha 1-AT, and PAI-1

Moreover, in contrast to LP/SD viral inactivation of biological fluids, viral inactivation of single donations or MP/SD of biological fluids avoids mechanical phenomena, such as aggregation, complexation, or oxidation phenomena, which all denature proteins.

Another advantage of the treatment of small quantities is that a tailor-made production of virally inactivated biological fluid is possible. Accordingly, a patient could for example be treated with virally inactivated plasma fractions coming exclusively from one donor, or small numbers of donors therefore decreasing the risks of exposure to any plasma-borne infectious agents.

In order to more fully illustrate the nature of the invention and the manner of practising the same, the following non-limiting examples are presented.

### EXAMPLES

### EXAMPLE 1: Viral inactivation of plasma with 1% TnBP and 1% Triton X-45

### Preparation of plasma

Apheresis plasma is collected in units of 250 ml and processed at the Shabrawishi hospital blood bank (Giza, Cairo, Egypt) from regular volunteer plasma donors. The plasma is frozen at -20°C or colder within 4 hours of collection and stored for less than 3 months prior to processing. The plasma donations are thawed in their collection bags in a temperature controlled water-bath at 30 +/- 1°C. For each batch, two units of plasma are pooled together (500 ml) in a transfer bag (JMS Singapore PTE LTD, Singapore), mixed for 5 min, and then split into two equal units (250 ml) that are transferred into individual polyvinyl chloride JMS transfusion bags.

In total, six batches of plasma are prepared.

### Viral inactivation

One of the two units of each of the six batches of plasma was virally inactivated as follows. The other unit was used for the viral inactivation according to the comparative example (*vide infra*).

The content of the plasma bag is treated inside the bag with 1% (w/w) TnBP (Prolabo, VWR, Fontenay-sous-bois, France) and 1% (w/w) Triton X-45 (Sigma Chemical, St Louis, MO) at 31°C for 4 hours, under constant gentle stirring at 150 rpm using a shaker-incubator (Lab Therm LT-W, Kühner, Switzerland).

The plasma-SD mixture is then transferred into a second polyvinyl chloride JMS transfusion bag and 7.5% (v/v; final concentration) castor oil (Fluka Chemie GmbH, Buchs, Switzerland) is added. After agitation on the shaker incubator (150 rpm) for 20 to 30 min and decantation for 30 min at 20-25°C, the plasma layer is recovered by gravity into a third polyvinyl chloride JMS bag and the oil layer is discarded.

The oil extraction is repeated to ensure sufficient removal of TnBP (which is soluble in oil) and avoid potential interferences on the α2-AP assay.

The plasma is clarified by centrifugation at 3800 g for 30 min at 4°C (Jouan, St-Herblain, France). 8 ml aliquots of the plasma is then subjected to hydrophobic interaction chromatography (HIC) in Poly-Prep chromatography columns (Biorad, Hercules, CA) packed with 1.5 g of preparative C18, 125 Å, 55-105 µm bulk material (Waters Corporation, Milford, MA). The plasma and the C18 material are mixed on a Baxter shaker (Baxter Healthcare Corp, Deerfield, USA) for 20 min at 20-25°C. Plasma is then recovered in the breakthrough fraction from the column.

### Sampling

Five 1 ml plasma samples are taken at the following stages of the process:
- after pooling the two plasma donations (start),
- after SD-treatment (before transfer in second plasma bag) (SD),
- after second oil extraction (oil),
- after centrifugation (centr.)
- after HIC (HIC).

The samples are kept on the bench at 20-25°C and frozen at -80°C at the end of each run. Plasma samples are therefore subjected to one freeze/thaw cycle, as occurs for the product that is transfused into a patient, prior to functional assays.

### Alpha-2-antiplasmin kinetic amidolytic assay

One sample of each stage is thawed at 37°C and analyzed within two hours. The α2-AP activity of standard human plasma (Dade-Behring, Newark, USA) and the above samples is determined by a kinetic amidolytic assay using Berichrom alpha 2-antiplasmin (Dade-Behring, Newark, USA) as described by the manufacturer in the leaflet (edition November 2003). The assay is based on the principle that the α2-AP in the sample deactivates the plasmin present. The residual plasmin content is determined in a kinetic test measuring the increase in absorbance at 405 nm (p-nitroaniline) according to the following reaction scheme:

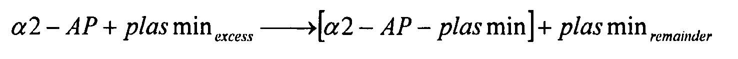

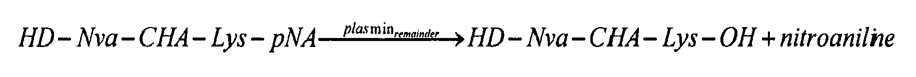

(HD-Nva-CHA-Lys-pNA = D-Norvalyl-cyclohexylalanyl-lysyl-p-nitroanilide).

20µl of plasma are mixed with 1000 µl of plasmin at 0.1 CTA (Committee for thrombolytic agents)/ml reconstituted in an aqueous buffer solution of 100 mmol/L potassium phosphate, 9 g/L sodium chloride, and 250g/L glycerol, pH 7.5, as indicated in the manufacturer's leaflet. After incubation for 1 min at 37°C, 100 µl of the D-Norvalyl-cyclohexylalanyl-lysil-p-nitroanilide substrate (Dade-Behring, Newark, USA) is added and samples were mixed carefully. The optical density at 405 nm of each sample is measured within 30 seconds using a Spinlab spectrophotometer (Spinreact S.A., Sant Esteve de Bas, Girona, Spain) and thereafter at exact 60 and 120 seconds intervals at 37°C to calculate the ΔOD/min and determine the α2-AP activity.

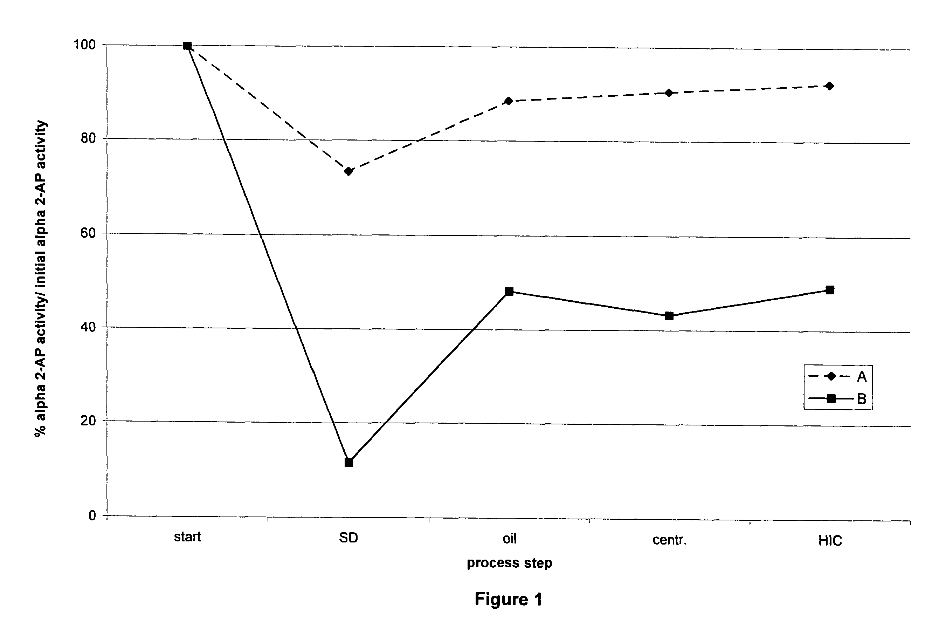

The results are shown in table 1. Graph A of Figure 1 reflects the evolution of the average α2-AP activity at each stage.

**Table 1: α2-AP activity**

| **Batch N°** | **α2-AP activity (%)** | | | | |
|---|---|---|---|---|---|
| | **Start** | **SD** | **Oil** | **centr.** | **HIC** |
| **1** | 95.5 | 76.2 | 90.0 | 88.0 | 92.5 |
| **2** | 111.0 | 65.8 | 82.2 | 85.9 | 103.8 |
| **3** | 107.9 | 75.2 | 84.6 | 90.2 | 92.5 |
| **4** | 115.0 | 90.3 | 104.1 | 107.5 | 101.8 |
| **5** | 109.2 | 95.3 | 105.6 | 105.6 | 96.0 |
| **6** | 03.8 | 68.5 | 107.0 | 103.8 | 105.3 |
| **average** | 107.1 | 78.6 | 94.7 | 96.8 | 98.7 |
| **mean %*** | | 73.4 | 88.4 | 90.4 | 92.1 |

| | | | | | |
|---|---|---|---|---|---|
| * of the initial α2-AP activity | | | | | |

### TnBP and Triton X-45 assays

Residual TnBP and Triton X-45 at the following stages
- after second oil extraction (oil),
- after centrifugation (centr.), and
- after HIC (HIC)
is determined from 1ml plasma samples and analyzed by gas chromatography and high performance liquid chromatography, as described in Burnouf et al., Transfusion 2006, 2100-8 and Burnouf et al., Vox Sang 2006, 91: 56-62*.*

Residual TnBP and Triton X-45 content of plasma is 50-100ppm after two oil extractions and after centrifugation, and <10ppm after C18 HIC.

### COMPARATIF EXAMPLE 1: Viral inactivation of plasma with 1% TnBP and 1% Triton X-100

The second unit of each of the six plasma batches prepared in Example 1 is virally inactivated as described above, except that 1% (v/v) Triton X-100 (Merck, Darmstadt, Germany) is used instead of Triton X-45.

Plasma samples are taken as described in Example 1 and α2-AP activity and TnBP and Triton X-100 contents are also determined as described in Example 1.

Table 2 shows the results of the α2-AP activity assay. Graph B of Figure 1 reflects the evolution of the average α2-AP activity at each stage.

**Table 2: α2-AP activity**

| **Batch N°** | **α2-AP activity (%)** | | | | |
|---|---|---|---|---|---|
| | **start** | **SD** | **oil** | **centr.** | **HIC** |
| **1** | 95.5 | 3.1 | 59.5 | 53.4 | 51.0 |
| **2** | 111.0 | 1.7 | 51.4 | 52.3 | 63.2 |
| **3** | 107.9 | 6.0 | 58.6 | 51.2 | 52.2 |
| **4** | 115.0 | 31.7 | 43.6 | 40.5 | 48.2 |
| **5** | 109.2 | 25.9 | 58.8 | 45.5 | 59.0 |
| **6** | 103.8 | 6.3 | 37.5 | 34.7 | 41.4 |
| **average** | | 12.5 | 51.6 | 46.3 | 52.5 |
| **mean %*** | | 11.6 | 48.2 | 43.2 | 49.0 |

| | | | | | |
|---|---|---|---|---|---|
| * of the initial α2-AP activity | | | | | |

Residual TnBP in plasma is between 20 and 70 ppm after 2 oil extractions and centrifugation, and less than 10ppm after C18-HIC, and Triton X-100 is still high (400-600 ppm) after oil extractions and centrifugation, and decrease (5-15 ppm) after the C18-HIC

From the above Examples it can clearly be seen that the significant loss of α2-AP activity in TnBP/Triton X-100 treated plasma is due to the presence of Triton X-100 and that the use of Triton X-45 (polyoxyethylene (4-5) p-t-octyl phenol (t-Oct-C₆H₄- (OCH₂CH₂)ₓOH, x= ~5)) as detergent in SD viral inactivation of biological fluids such as plasma does not lead to a significant loss of α2-AP activity.

### COMPARATIF EXAMPLE 2: Treatment of plasma without process chemicals

In this example one batch of plasma is prepared as described in Example 1.

One of the two units of the batch is processed as described in Example 1 with the exception that no process chemicals (solvent, detergent) are added to the plasma.

Plasma samples are taken as described in Example 1 and α2-AP activity are also determined as described in Example 1.

The α2-AP activity of the starting material is 98.8 %. It is 95.8 % after the incubation step, 101.2 % after the second oil extraction, 98.0 % after centrifugation and 101.8 after HIC.

This example clearly demonstrates that essentially full α2-AP activity was observed when performing the process steps of SD viral inactivation without solvent/detergent addition. It may thus be concluded that the various process steps do not affect α2-AP activity.

## Claims

1. Use of polyoxyethylene (4-5) p-t-octyl phenol (t-Oct-C₆H₄-(OCH₂CH₂)ₓOH, x= ~5) or polyoxyethylene (20) sorbitan monooleate in solvent/detergent viral inactivation of biological fluids for the preparation of a virally inactivated biological fluid **characterized in that** the virally inactivated biological fluid has an alpha-2-antiplasmin (α2-AP) activity of at least 85 % of the initial alpha-2-antiplasmin activity.

2. The use of Claim 1, **characterized in that** the virally inactivated biological fluid has an alpha-2-antiplasmin content of at least 88 %, preferably 90 % y, and even more preferably at least 92 % of the initial alpha-2-antiplasmin activity.

3. The use according to Claim 1 or 2, **characterized in that** the virally inactivated plasma has an alpha-1-antitrypsin (α1-AT) activity of at least 80 %, preferably at least 85 %, and even more preferably of 90 % of the initial alpha-1-antitrypsin activity.

4. The use according to any of Claims 1 to 3, **characterized in that** the virally inactivated plasma has a plasma activator inhibitor-1 (PAI-1) activity of at least 80 %, preferably at least 85 %, and even more preferably of 90 % of the initial alpha-1-antiplasmin activity.

5. The use according to any of Claims 1 to 4, **characterized in that** the polyoxyethylene (4-5) p-t-octyl phenol is employed at a concentration of 0.1 to 2.0 %, preferably 0.3 to 1.5 %, and even more preferably 0.5 to 1% by weight with respect to the weight of the biological fluid.

6. The use according to any of Claims 1 to 5, **characterized in that** the polyoxyethylene (20) sorbitan monooleate is employed at a concentration of 0.1 to 2.0 %, preferably 0.3 to 1.5 %, and even more preferably 0.5 to 1% by weight with respect to the weight of the biological fluid.

7. The use according to any of Claims 1 to 6, **characterized in that** the biological fluid is selected from the group consisting of mammalian blood, blood plasma, blood serum, plasma fractions, precipitates from blood fractions and supernatants from blood fractionation, platelet poor plasma, cryo-poor plasma (cryosupernatant), recombinant products, and transgenic products.

8. The use according to any of Claims 1 to 6, **characterized in that** the solvent is selected within the group consisting of tri-(n-butyl)phosphate (TnBP), tri-(t-butyl)phosphate, tri-(n-hexyl)phosphate, tri-(2-ethylhexyl)phosphate, tri-(n-decyl)phosphate, di-(n-butyl)phosphate, di-(t-butyl)phosphate, di-(n-hexyl)phosphate, di-(2-ethylhexyl)phosphate, di-(n-decyl)phosphate, ethyl di(n-butyl) phosphate and mixtures thereof.
